Europäisches Patentamt

(19) **European Patent Office**

**Office européen des brevets**

(11) Veröffentlichungsnummer : **0 002 776**
**B2**

(12) **NEUE EUROPÄISCHE PATENTSCHRIFT**

(45) Veröffentlichungstag der neuen Patentschrift :
04.03.87

(21) Anmeldenummer : 78101751.2

(22) Anmeldetag : 18.12.78

(51) Int. Cl.⁴ : **A 61 M   5/14**, G 06 F **15/42**

(54) **Vorrichtung zur vorprogrammierbaren Insulin-Infusion.**

(30) Priorität : **28.12.77 DE 2758467**

(43) Veröffentlichungstag der Anmeldung :
**11.07.79 Patentblatt 79/14**

(45) Bekanntmachung des Hinweises auf die Patenterteilung : **29.04.81 Patentblatt 81/17**

(45) Bekanntmachung des Hinweises auf die Entscheidung über den Einspruch : **04.03.87 Patentblatt 87/10**

(84) Benannte Vertragsstaaten :
**CH DE FR GB SE**

(56) Entgegenhaltungen :
DE-A- 2 513 467
US-A- 4 003 379
**"Normalization of Insulin Delivery to Diabetics by Pulsed Insulin Infusion" von Paul Martin und Saul Genuth in IEEE Transactions on Biomedical Engineering, vol. BME-24, no. 2, März 1977, Seiten 116-121**

(73) Patentinhaber : **Siemens Aktiengesellschaft Berlin und München**
**Wittelsbacherplatz 2**
**D-8000 München 2 (DE)**

(72) Erfinder : **Franetzki, Manfred, Dr.**
**Schleifweg 7b**
**D-8521 Uttenreuth (DE)**
Erfinder : **Gagneur, Klaus**
**Wiesenweg 11**
**D-8521 Bubenreuth (DE)**
Erfinder : **Prestele, Karl**
**Leipziger Strasse 74**
**D-8520 Erlangen (DE)**

Jouve. 18, rue St-Denis, 75001 Paris, France

## Beschreibung

Die Erfindung bezieht sich auf eine Vorrichtung zur vorprogrammierbaren Insulininfusion bei der Diabetes-Therapie des menschlichen oder tierischen Körpers, bestehend aus einer Mikrodosiereinheit für das Insulin sowie einer damit in Verbindung stehenden, einen Programmgeber für die Mikrodosiereinheit aufweisenden Steuervorrichtung. Dabei kann die Mikrodosiereinheit für die Flüssigkeit zusammen mit der Steuervorrichtung oder separat im Körper implantierbar oder auch extern an der Körperoberfläche tragbar sein.

Bei der Diabetes-Therapie ist es erwünscht, Insulin fortwährend in unterschiedlichen Raten in den Körper des Patienten zu infundieren, weil der Insulinbedarf des Zuckerkranken während des Tages, bedingt z. B. durch den Rhythmus der Mahlzeiten, großen Schwankungen unterworfen ist. Es hat sich gezeigt, daß — wenn keine mittels Glucosesensor selbsttätig regelnden Infusionsgeräte zur Verfügung stehen — die Insulinabgabe am günstigsten nach einem für den Patienten individuell einstellbaren und vorprogrammierbaren Tagesprofil erfolgen sollte (z. B. DE-OS 24 51 424).

Vorrichtungen zur programmierten Infusion, z. B. nach der DE-OS 25 13 467, könnten in der Weise weitergebildet werden, daß das Steuerprogramm auf einem externen Programmiergerät entsprechend dem 24-stündigen Tagesablauf in diskreten Zeitschritten einprogrammiert wird, wobei die Steuervorrichtung lediglich zur Übernahme des vorprogrammierten Steuerprogramms mit dem Programmiergerät bzw. einem Programmträger elektrisch verbunden wird. Eine derartige Vorrichtung hat den Vorteil, daß die Vorprogrammierung des Tagesprofils durch den Arzt in einfacher und übersichtlicher Weise erfolgen kann ; beispielsweise lassen sich solche Vorrichtungen dann anwenden, wenn innerhalb einer längeren Untersuchungsperiode, während der sich der Patient unter ärztlicher Kontrolle im Krankenhaus befindet, ein optimales Tagesinfusionsprofil für eine nachfolgend komplett zu implantierende Infusionseinrichtung ermittelt werden soll. Allerdings hat in diesem Fall der Patient in der Folgezeit im allgemeinen keinen direkten Zugriff zur Veränderung der vorprogrammierten Dosierung und ist demzufolge an einen relativ regelmäßigen Tagesablauf mit zeitlich und mengenmäßig vorbestimmter Einnahme seiner Mahlzeiten gebunden, da nur ein eingestelltes Steuerprogramm im Programmspeicher abgespeichert ist.

Weiterhin ist aus der Zeitschrift « IEEE Transactions on Biomedical Engineering », Vol. BME-24, No. 2 (1977), S. 116 bis 121, ein Gerät der eingangs genannten Art bekannt, mit dem eine programmierte Insulinverabreichung durchführbar ist. Dem Infusionsprogramm liegt jeweils eine an den individuellen Bedarf angepaßte Infusionsdosis zugrunde, wozu mathematische Modellrechnungen durchgeführt werden, mit denen für eine Mahlzeit zur Anpassung an die physiologischen Verhältnisse spezifische e-Funktionen für den Infusionsratenanstieg einerseits und den Infusionsratenabfall andererseits ermittelt werden. Die Parameter der Exponentialfunktionen beinhalten eine Körpergewichtskonstante und eine Insulinabbaukonstante, welche beide individuell für den betreffenden Patienten festgelegt werden müssen. An einem Satz von Potentiometern und Schaltern, die man bei abstrahierender Auslegung als Programmgeber des Gerätes ansehen kann, sind vier Variable einzustellen.

Ein gesamtes Tagesprogramm der Infusion besteht nach dieser Literaturstelle aus drei Dosierungsmaxima mit jeweils exponentiellem Verlauf, welche den Mahlzeiten (Frühstück, Mittagessen, Abendessen) zugeordnet sind und einen Abstand von etwa 5 Stunden haben. Diese Zeit wird nach den erwähnten Modellrechnungen benötigt, um die Infusionsrate vom Basiswert auf den Spitzenwert relativ rasch ansteigen und sodann vom Spitzenwert auf den Basiswert verlangsamt abklingen zu lassen. Die Spitzenprofile sind auf eine einstellbare Basalrate aufgesetzt. Vor jeder einzelnen Mahlzeit wird das Spitzenprofil durch entsprechende Wahl der Parameter eingestellt.

Nach Ermittlung der patientenindividuellen Parameter für die Infusionsfunktion wird also bei diesem Stand der Technik dreimal pro Tag ein Mahlzeitenprogramm der Insulinverabreichung mit einem Infusionsratenverlauf nach e-Funktionen aufgestellt. Durch Integration der Infusionsrate über einen Zeitraum von 24 Stunden ist es möglich, die Tagesdosis in Insulineinheiten zu ermitteln.

Bei dem vorbekannten Gerät müssen die vier Parameter, welche den programmierten Infusionsratenverlauf und damit die tatsächliche Insulindosis beeinflussen, vor einer jeden Mahlzeit von medizinischem Fachpersonal am Programmgeber (Potentiometer, Schalter) variiert werden. Damit ist in der Krankenhausroutine ein relativer starrer Tagesablauf bezüglich der Einnahme der Mahlzeiten vorgezeichnet. In diesem Zusammenhang wäre es zwar auch möglich, ein automatisches Programm zum Verändern der Infusionsrate durch den Patienten selbst zu starten, so daß unter bestimmten Voraussetzungen eine größere Flexibilität bei der Einnahme der Mahlzeiten erreicht werden würde. Vorher wäre aber wieder die Einstellung der vier Programmparameter notwendig. Hierzu bedarf es aber der Überwachung und Mithilfe durch den Arzt. Im Ergebnis kann also dieses vorbekannte Gerät nur unter medizinischer Kontrolle mit weitgehend vorgegebenem Tagesablauf angewendet werden, da bei einer Programmänderung zu jeder Mahlzeit der Programmablauf jedesmal neu mittels der Potentiometer und Schalter von Hand eingestellt werden muß.

Eine ständige medizinische Kontrolle ist jedoch in der Praxis nicht realisierbar, so daß die Bindung an ein Festprogramm angezeigt ist. Bei bestimmten Fällen des Diabetes wäre jedoch die Bindung des Patienten an den regelmäßigen Tagesablauf nicht unbedingt notwendig. Von der Konstitution des Patienten her wäre es möglich, daß der Patient einem normalen, einem Nichtdiabetiker entsprechenden Tagesablauf nachgehen kann, sofern ihm nur bei den Mahlzeiten die notwendigen Insulinmengen in entsprechender Zeitrelation verabreicht werden. In einem solchen Fall sind also die starre Bindung des Patienten an das Tagesprofil der vorprogrammierten Insulininfusion sowie die ständige medizinische Kontrolle überflüssig und können auf Dauer für den Patienten sogar zur Belastung werden.

Aufgabe der Erfindung ist es daher, ein Gerät der eingangs genannten Art so auszugestalten, daß es einerseits eine praxisgerechte, vorprogrammierte Infusion ermöglicht und andererseits dem Patienten weitgehende Freiheit bei der zeitlichen Einnahme der Mahlzeiten und der Zusammensetzung derselben einräumt. Der programmäßige Ablauf der Infusion soll demgemäß dem tatsächlichen Insulinbedarf soweit wie möglich entsprechen, wobei das Gerät vom Patienten selbst ohne spezielle Fachkenntnis handhabbar sein soll.

Diese Aufgabe wird bei einer Vorrichtung der eingangs genannten Art erfindungsgemäß dadurch gelöst, daß der Programmgeber aus einem der Steuervorrichtung zugeordneten Programmspeicher besteht, in welchem mehrere verschiedene Steuerprogramme gleichzeitig abgespeichert sind, die durch direkte Eingabe der den mit einer Mahlzeit aufgenommenen Broteinheiten entsprechenden Anzahl Insulineinheiten mittels einer Eingabeeinheit vom Patienten anwählbar sind, und daß mit dem Anwahlvorgang an der Eingabeeinheit der programmäßige Ablauf der Insulininfusion gestartet wird.

Mit der Erfindung ist nun also eine Möglichkeit geschaffen, die Vorteile einer Vorrichtung zur vorprogrammierten Insulininfusion mit der Möglichkeit einer zeitlich und mengenmäßig variablen Abgabe von Infusionsflüssigkeit zu verbinden.

Der Patient wählt lediglich bei einer Mahlzeit entsprechend den mit der Mahlzeit aufzunehmenden Broteinheiten eine äquivalente Menge an Insulineinheiten auf der z. B. von ihm getragenen Steuervorrichtung an, wobei damit unmittelbar ein bestimmtes vorprogrammiertes Steuerprogramm aus dem Programmgeber abgerufen wird. Die Broteinheiten einer Mahlzeit sind direkt mit der in der Nahrung enthaltenen Menge an Kohlehydraten korreliert und dem Diabetiker üblicherweise für jeden Bestandteil seiner Nahrung bekannt.

Im Gegensatz zu der aus der Literatur vorbekannten Vorrichtung zur vorprogrammierbaren Insulininfusion muß bei dem Gerät nach der Erfindung der Patient nicht unter ständiger Kontrolle von medizinischem Fachpersonal stehen. Es ist nicht notwendig, vor den einzelnen Mahlzeiten eine Mehrzahl von Parametern, die die Infusionsrate wechselseitig beeinflussen, einzustellen und zu optimieren. Vielmehr braucht lediglich vom Patienten die gesamte, für eine Mahlzeit notwendige Dosis an Insulin, also eine einzige Größe, angewählt zu werden, worauf der programmäßige Ablauf ohne weitere Einstellung von Funktionsparametern erfolgt. Damit ist ein Gerät für den ambulanten Langzeitbetrieb bereitgestellt, das vom Diabetiker handhabbar und ohne wissenschaftliche Spezialkenntnisse beherrschbar ist.

In vorteilhafter Ausgestaltung der Erfindung wird an der Steuervorrichtung eine zeitlich konstante Basisrate (Basalrate) eingestellt, der dann bei den Mahlzeiten entsprechend dem eingespeicherten Steuerprogramm die zusätzlichen Insulinmengen jeweils als sogenannter Bolus aufgesetzt sind. Die Ratenprofile können dabei Rechteckform besitzen, wobei entweder die Abgabezeit oder die Rate als konstant vorgegeben ist. Der Flächeninhalt dieser Rechtecke entspricht der abgegebenen Insulinmenge. Alternativ dazu können aber auch durch Einprogrammierung von Rechtecken mit sowohl veränderlicher Höhe als auch veränderlicher Breite bei Anwahl einer n-fachen Insulinmenge jeweils Infusionszeit und Rate gleichzeitig im entsprechenden Verhältnis verändert werden.

In anderer vorteilhafter Ausbildung der Erfindung entspricht das Steuerprogramm der Infusionsrate im wesentlichen einer Dreiecksform, beispielsweise einer rechtwinkligen Dreiecksform wobei die Infusionsrate von einem Basiswert schnell auf den Höchstwert und anschließend vom Höchstwert mit vorgegebener Funktionaler Abhängigkeit wieder auf den Basiswert abnimmt. Der Abnahmegrad ist dabei linear mit vorgegebener Steigung. Er kann aber auch exponentiell nach Art einer e-Funktion sein. Die Infusionsratenabgabe kann flächenmäßig natürlich auch komplizierter aufgebaut und der tatsächlichen Insulinabgabe eines natürlichen Pankreas angepaßt sein, wobei bei Vorgabe eines Profils mit nichtlinearen funktionalen Abhängigkeiten ein Rechner (Mikroprozessor) zur Anpassung der angewählten Infusionsmenge an das Abgabeprofil vorzusehen ist.

Die mit der Erfindung geschaffene Infusionsvorrichtung besteht vorzugsweise aus zwei Einzelgeräten. Dabei kann die Mikrodosiereinheit fakultativ als Implantat mit transkutaner Signalübertragung von der extrakorporal zu tragenden Steuervorrichtung oder zusammen mit der Steuervorrichtung als extrakorporal anzuordnendes Gerät ausgebildet sein. Im letzteren Fall ist dann eine direkte Leitungsverbindung zur Signalübertragung zwischen den beiden Geräten möglich.

Weitere Vorteile und Einzelheiten der Erfindung ergeben sich aus der nachfolgenden Figurenbeschreibung von Ausführungsbeispielen anhand der Zeichnung und in Verbindung mit den weiteren Unteransprüchen.

Es zeigen :

Figur 1 ein Prinzipschaubild von Steuervorrichtung und Mikrodosiereinheit mit Blockdarstellung der einzelnen Funktionseinheiten,

Figur 2 eine Außenansicht einer Steuervorrichtung in perspektivischer Darstellung, und

Figur 3 verschiedene Impulsdiagramme a bis e für mögliche programmäßige vorgegebene Infusionsraten der Insulinabgabe.

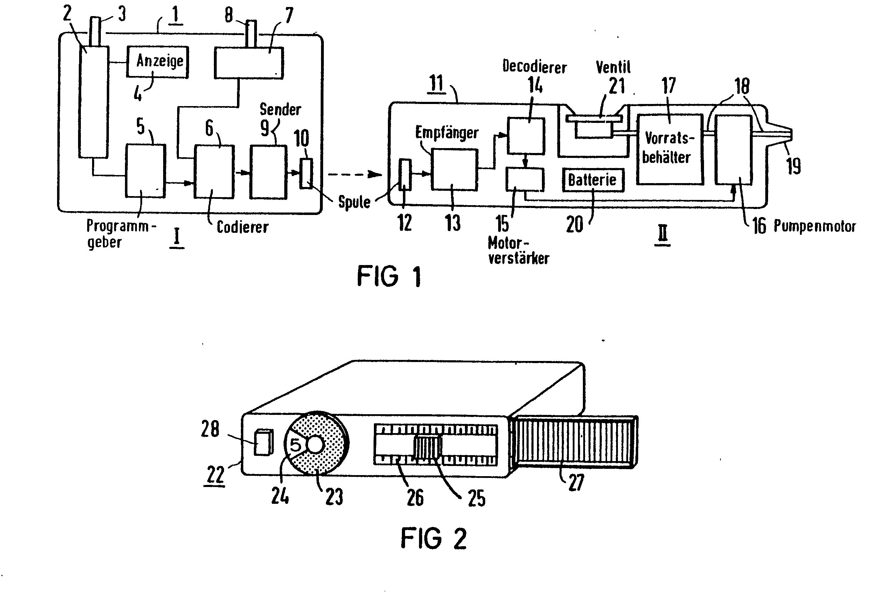

In der Fig. 1 ist mit I eine Steuervorrichtung und II eine Mikrodosiereinheit bezeichnet. Die Steuervorrichtung I besteht aus einem Gerätegehäuse 1 von der Größenordnung eines Taschenrechners, welches die elektronischen Bauteile aufnimmt und von außen mittels mehrerer Bedienelemente anwählbar ist. In einer Eingabeeinheit 2 wird über ein Betätigungsglied 3 die Anzahl der abzugebenden Insulineinheiten angewählt, welche am Gerätegehäuse 1 an einer Anzeigeeinheit 4 sichtbar sind. Von der Eingabeeinheit 2 wird ein Kurzprogrammgeber 5 angesteuert, in dem programmabläufe zwischen etwa 0,5 und 3 Stunden für die Infusionsrate gespeichert sind, welche durch Abruf mit der Eingabe der abzugebenden Insulineinheiten gestartet werden. Als Programmgeber 5 werden beispielsweise digitale Halbleiterspeicher, sog. RAM's, verwendet, die für beliebige Programmabläufe programmierbar sind. Vom Programmgeber 5 wird ein Signalcodierer 6 angesteuert, der das angewählte Programm zur Signalübertragung entsprechend aufbereitet. Der Signalcodierer 6 ist weiterhin von einem Konstantratengeber 7 mit Betätigungsglied 8 ansteuerbar. Vom Signalcodierer 6 werden die codierten Signale auf einen Sender 9 gegeben, mit dem zwecks Fernsteuerung eine elektromagnetische Spule 10 angesteuert wird.

Im Ausführungsbeispiel sollen die Steuersignale drahtlos, und zwar induktiv, übertragen werden. Statt einer induktiven Signalübertragung sind natürlich andere elektromagnetische Signalübertragungsverfahren, beispielsweise Infrarot-, oder aber auch Ultraschallsignalübertragungsverfahren möglich. Alternativ dazu kann bei extrakorporaler Anordnung der Mikrodosiereinheit die Steuervorrichtung auch über eine direkte elektrische Leitungsverbindung an die Mikrodosiereinheit angekoppelt sein.

Das Gehäuse der Mikrodosiereinheit II ist mit 11 bezeichnet. Es enthält in Analogie zur Steuervorrichtung I eine Empfängerspule 12 mit nachgeschaltem elektronischen Empfänger 13 und Decodierer 14. Über den Decodierer 14 wird ein Motorverstärker 15 angesteuert, mit dem der Antriebsmotor einer mechanischen Pumpe 16 angetrieben wird. Mittels Pumpe 16 wird aus einem Vorratsbehälter 17 das flüssige Insulin über eine Verbindungsleitung 18 zu einem Katheteranschluß 19 am Gerätegehäuse 11 befördert. Das Gehäuse 11 der Mikrodosiereinheit I enthält weiterhin noch eine Batterie 20 als Energiequelle für den Pumpenantrieb sowie ein Nachfüllventil 21, über das mittels Spritze über eine sich selbsttätig schließende Membran, — bei implantierter Mikrodosiereinheit gegebenenfalls transkutan — Insulin in den Vorratsbehälter 17 nachgefüllt werden kann.

In der Fig. 2 ist das Gehäuse einer Steuervorrichtung mit 22 bezeichnet. An seiner Seitenfläche befindet sich ein Drehschalter 23, mit dem auf einer Wählscheibe 24 diskrete Infusionsmengen in Insulineinheiten (z. B. in Schritten von 1 bis 10 IE) angewählt werden können, die in den Programmgeber eingegeben werden. Daneben ist ein Schiebeschalter 25 angeordnet, mit dem auf einer Skala konstante Basisraten der Insulinabgabe (z. B. einstellbar zwischen 0,4 und 2 IE/h) eingestellt werden können. Da die Basisrate im allgemeinen nur einmal vorgegeben wird und dann über einen längeren Zeitraum nicht mehr verändert werden soll, wird die Skala 26 im Betriebszustand des Steuergerätes von einer Klappe 27 abgedeckt.

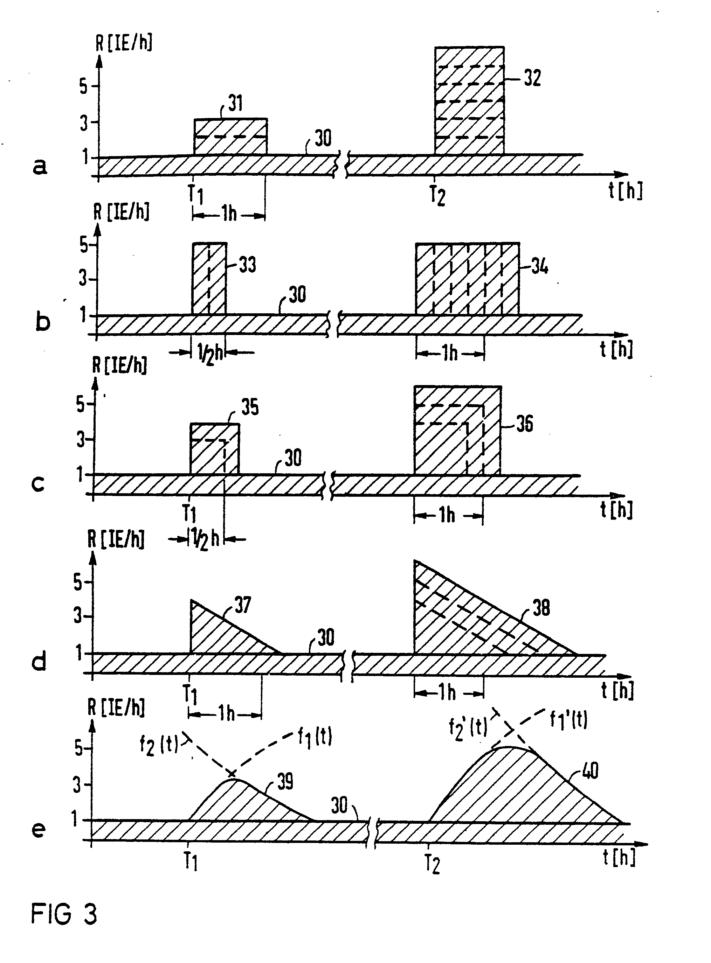

In den Impulsdiagrammen nach Fig. 3 ist die Flußrate der Mikrodosiereinheit II für verschiedene Kurzprogramme als Funktion der Zeit dargestellt. Die zur Abszisse parallelen Geraden 30 bedeuten dabei jeweils, daß eine konstante Basisrate der Abgabe von Insulineinheiten, nämlich 1 IE/h eingestellt ist. Im Impulsdiagramm a steigt nun die Abgaberate beim Anwählzeitpunkt $T_1$ von 1 IE/h auf 3 IE/h und fällt nach einer Stunde wieder auf den Ausgangswert. Zum Anwählzeitpunkt $T_2$ steigt die Abgaberate auf eine Infusionsrate von 7 IE/h und fällt nach einer Stunde wieder auf den Ausgangswert. Die von der Abgaberate aufgespannten Rechtecken 31 bzw. 32 entsprechen jeweils einer Gesamtinsulinabgabe von 2 IE bzw. 6 IE. Für eine derartige Abgabe ist also lediglich im Programmgeber 5 die Abgabezeit von einer Stunde gespeichert. Der Patient braucht dann nur noch gemäß seiner Mahlzeit am Schalter 23 die erforderlichen Insulineinheiten anzuwählen, wonach sich die Abgaberate für die vorgegebene Zeitdauer ändert.

Im Impulsdiagram b ist dagegen die Abgaberate konstant vorgegeben. Durch Anwählen der Insulineinheiten am Schalter 23 wird dann vom Programmgeber die Zeitdauer der Insulinabgabe bestimmt, die von z. B. 0,5 bis 3 Stunden veränderbar ist. Die Rechtecke 33 bzw. 34 bedeuten in diesem Fall wieder 2 IE bzw. 6 IE.

Im Impulsdiagram c wird ein Elementarrechteck mit den Kantenlängen 1 = 0,5 h und I = 2 IE/h aufgebaut. Ein derartiges Rechteck ist im Programmgeber 5 bei entsprechender Normierung als Quadratspeicherbar. Bei Verdoppelung eines solchen normierten Elementarquadrates verändern sich die Kantenlängen jeweils im Verhältnis $\sqrt{2} : 1$. Die Quadrate 35 bzw. 36 bedeuten damnach wiederum 2 IE bzw. 6 IE. Eine Vervielfachung der Insulinmenge um den Faktor n ergibt demnach eine Veränderung der Abgabezeit und Abgaberate um den Faktor $\sqrt{n}$.

Im Impulsdiagram d wird zum Zeitpunkt $T_1$ die Abgaberate vom Basiswert 1 IE/h auf einen Wert von 4 IE/h angehoben und fällt von dort linear mit vorgegebener Steigung auf den Ausgangswert zurück. Zum Zeitpunkt $T_2$ wird die Abgaberate auf einen Wert von 6 IE/h angehoben, von wo sie mit der gleichen vorgegebenen Steigung ebenfalls wieder auf den Ausgangswert zurückfällt. Auf die konstante Basisrate werden also in diesem Fall jeweils kongruente Dreiecke aufgesetzt, wobei dem Dreieck 37 wiederum 2 IE und dem Dreieck 38 6 IE entsprechen. Die Abgaberaten und -zeiten verändern sich demzufolge bei dreifacher Abgabe als Dreieckskatheten im Verhältnis $\sqrt{3}$, wobei die Steigung der Dreieckshypotenuse im Programmgeber eingespeichert ist. Statt der Geraden kann auch eine e-Funktion für den Abfall der Infusionsrate vom Höchstwert auf den Basiswert vorgesehen sein. Die Insulinabgabe in Dreiecksform hat insbesondere den Vorteil, daß der Übergang von erhöhter Abgaberate zur Normal-Basisrate nicht spontan, sondern stetig — d. h. zeitlich kontinuierlich — erfolgt.

Analysiert man den tatsächlichen Insulinbedarf bei einer Mahlzeit und nachfolgender Verdauung im einzelnen, so erhält man entsprechend der Insulinabgabe eines natürlichen Pankreas zunächst ein schnelles Ansteigen auf einen Höchstwert mit aufgesetztem Peak und anschließendem langsamen Abfallen bis auf den Ausgangswert nach Art von e-Funktionen. Dieser tatsächliche Bedarf läßt sich allerdings nur durch mehrere Exponentialfunktionen anpassen, wobei die umschlossene Fläche dann natürlicherweise komplizierter zu erfassen ist. Ein derartiger Verlauf der Abgaberate ist im Impulsdiagramm e vereinfacht mit zwei Funktionen angepaßt dargestellt. Die Abgaberaten werden durch die Exponentialfunktionen $f_i^K(t)$ bestimmt wobei die freien Parameter beliebig anpaßbar sind. In einem solchen Fall muß dem Programmgeber allerdings ein Rechner, beispielsweise Mikroprozessor, zugeordnet werden, der die vom Patienten angewählte Infusionsmenge, d. h. einen angegebenen Flächeninhalt, an die entsprechende Fläche mit vorgegebenen Konturen gemäß der im Programmgeber gespeicherten funktionalen Abhängigkeit anpaßt.

Es hat sich gezeigt, daß es in bestimmten Enzelfällen — beispielsweise nach einer schwerverdaulichen Mahlzeit — notwendig sein kann, abweichend vom vorprogrammierten Kurzprogramm die Abgabezeit zu variieren. Im Impulsdiagramm nach Fig. 3d bedeutet dies beispielsweise, daß die Steigung der Geraden veränderbar ist. Zu diesem Zweck wird dann am Steuergerät nach Fig. 2 ein weiterer Stufenschalter 28 zur Zeitdehnung angeordnet, mit dem vom Patienten entsprechend Verdaulichkeit und Resorption einer eingenommenen Mahlzeit ein Zeitmaßstab des Programms einstellbar ist.

**Patentansprüche**

1. Vorrichtung zur vorprogrammierbaren Insulininfusion bei der Diabetes-Therapie des menschlichen oder tierischen Körpers, bestehend aus einer Mikrodosiereinheit (II) für das Insulin sowie einer damit in Verbindung stehenden, einen Programmgeber (5) für die Mikrodosiereinheit (II) aufweisenden Steuervorrichtung (I), dadurch gekennzeichnet, daß der Programmgeber (5) aus einem der Steuervorrichtung (I) zugeordneten, Programmspeicher besteht, in welchem mehrere verschiedene Steuerprogramme gleichzeitig abgespeichert sind, die durch direkte Eingabe der den mit einer Mahlzeit aufgenommenen Broteinheiten (BE) entsprechenden Anzahl Insulineinheiten (IE) mittels einer Eingabeeinheit (2) vom Patienten anwählbar sind, und daß mit dem Anwählvorgang an der Eingabeeinheit (2) der programmäßige Ablauf der Insulininfusion gestartet wird.

2. Vorrichtung nach Anspruch 1, dadurch gekennzeichnet, daß an der Steuervorrichtung (I) von Patienten zusätzlich zum programmäßigen Ablauf (31 bis 40) einer Abgabe von angewählten Insulineinheiten (IE) eine zeitlich konstante Basisrate (30) der Abgabe von Insulineinheiten (IE/h) einstellbar ist.

3. Vorrichtung nach Anspruch 1, dadurch gekennzeichnet, daß mit Anwählen der abzugebenden Insulineinheiten (IE) ein solches Abgabeprofil vom Programmspeicher (5) abgerufen wird, das genaü an die tatsächliche Insulinabgabe eines naturlichen Pankreas angepasst ist.

4. Vorrichtung nach Anspruch 1, 2 oder 3, dadurch gekennzeichnet, daß das Steuerprogramm des Programmgebers (5) Rechteckform (31 bis 34) aufweist, wobei eine der Rechteckkanten vom Patienten als Infusionsrate bzw. Infusionszeit vorwählbar ist und die andere Rechteckkante zur Konstanthaltung der Dosierungsrate entsprechend dem vorgegebenen Steuerprogramm von Programmgeber (5) als variable Breite bzw. variable Amplitude des Rechtecks (31 bis 34) bestimmt wird (Fig. 3b).

5. Vorrichtung nach einem der Ansprüche 1 bis 4, dadurch gekennzeichnet, daß das Steuerprogramm des Programmgebers (5) Rechteckform aufweist, wobei die Seitenkanten eines eine abgegebene Insulinmenge symbolisierenden Elementarrechtecks der kleinsten vorgebbaren Infusionsrate bzw. -zeit entsprechen und die vom Patienten angewählte Dosierungsmenge an Insulineinheiten vom Programmgeber (5) als Vielfaches der Fläche des Elementarrechtecks bestimmt wird, so daß einer n-fachen Menge an Insulineinheiten (IE) um jeweils $\sqrt{n}$ größere Infusionsraten und -zeiten entsprechenden (Fig. 3c).

6. Vorrichtung nach einem der Ansprüche 1 bis 5, dadurch gekennzeichnet, daß das Steuerprogramm des Programmgebers (5) im wesentlichen einer Dreiecksform entspricht, wobei die Infusionsrate von einem Basiswert zum Höchstwert ansteigt und anschließend von diesem Höchstwert mit vorgegebener funktionaler Abhängigkeit wieder abfällt auf den Basiswert, insbesondere linear mit vorgegebener Steigung oder exponentiell nach Art einer e-Funktion.

7. Vorrichtung nach Anspruch 6, dadurch gekennzeichnet, daß das Steuerprogramm des Programmgebers eine rechtwinklige Dreiecksform (37, 38) aufweist, wobei die Fläche eines kleinsten Elementardreiecks, dessen Katheten der kleinsten vorgebbaren Infusionsrate bzw. -zeit entsprechen, einer Insulineinheit entspricht und die vom Patienten angewählte Dosierungsmenge an Insulineinheiten vom Programmgeber (5) als Vielfaches der Fläche des Elementardreiecks bestimmt wird, so daß bei einer n-fachen Menge von Insulineinheiten ein zum Elementardreieck kongruentes Dreieck mit um $\sqrt{n}$ längeren Katheten im Vergleich zu denen des Elementardreiecks aufgebaut werden (Fig. 3d).

8. Vorrichtung nach einem der vorhergehenden Ansprüche, dadurch gekennzeichnet, daß der Programmgeber (5) digitale elektronische Speicher enthält.

9. Vorrichtung nach Anspruch 1, dadurch gekennzeichnet, daß das Gerätegehäuse (1, 22) der Steuervorrichtung (I) Einstell- und Anzeigemittel (3, 4, 8, 23 bis 26) für die Eingabe und Anzeige der zu verabreichenden Insulineinheiten (IE) bzw. der aufgenommenen Broteinheiten (BE) aufweist.

10. Vorrichtung nach Anspruch 9, dadurch gekennzeichnet, daß die Steuervorrichtung einen Betätigungsschalter (28) zur Veränderung der mittels Steuerprogramm des Programmgebers (5) bestimmten Abgabezeit der Insulinverabreichung aufweist.

11. Vorrichtung nach Anspruch 1, dadurch gekennzeichnet, daß die Steuervorrichtung (I) in einem von der Mikrodosiereinheit (II) getrennten Gerätegehäuse (1) untergebracht ist.

12. Vorrichtung nach Anspruch 11, dadurch gekennzeichnet, daß zur Übertragung der Steuersignale zwischen Steuervorrichtung (I) und Mikrodosiereinheit (II) eine elektrische Leitungsverbindung vorgesehen ist.

13. Vorrichtung nach Anspruch 11, dadurch gekennzeichnet, daß die Steuersignale von der Steuervorrichtung (I) zur Mikrodosiereinrichtung (II) drahtlos mittels Fernsteuerung (10, 12), vorzugsweise induktiv, übertragen werden.

**Claims**

1. Device for the preprogrammable insulin infusion in diabetes therapy of human or animal bodies, consisting of a micro-dose unit (II) for the insulin and a control device (I) which is connected thereto and has a programmer (5) for the micro-dose unit (II), characterised in that the programmer consists of a programme store which is assigned to the control device (I) and in which a plurality of different control programmes are simultaneously stored, which can be selected by the patient by direct input of the number of insulin units (IE) which corresponds to the carbohydrate content (BE) absorbed at a meal time, with the aid of an input unit (2) ; and that the selection operation at the input device (2) serves to start the programme sequence of the insulin infusion.

2. Device as claimed in Claim 1, characterised in that the patient can set the control device (I), in addition to the programmed sequence (31 to 40) of a delivery of selected insulin units (IE) a base rate 30, constant in time, for the emission of insulin units (IE/h).

3. Device as claimed in Claim 1, characterised in that the selection of the insulin units (IE) which are to be dispensed results in an output profile from the programme store (5) which is precisely matched to the actual insulin output of a natural pancreas.

4. Device as claimed in Claims 1, 2 or 3, characterised in that the control programme of the programmer (5) has a rectangular characteristic (31 to 34), in which one of the rectangular edges can be preselected by the patient as the infusion rate or infusion time and the other rectangular edge, in order to maintain constant the dose rate in accordance with the predetermined control programme, is specified by the programmer (5) as a variable width or a variable amplitude respectively of the rectangle (31 to 34) (Fig. 3b).

5. Device as claimed in one of Claims 1 to 4, characterised in that the control programme of the programmer (5) has a rectangular characteristic in which case the side edges of an elementary rectangle which symbolises a quantity of output insulin correspond to the smallest predeterminable infusion rate or time, and the dose quantity of insulin units selected by the patient is specified by the programmer (5) as a multiple of the area of the elementary rectangle so that an n-multiple of the quantity of insulin units (IE) corresponds to infusion rates and times increased by $\sqrt{n}$ (Fig. 3c).

6. Device as claimed in one of Claims 1 to 5, characterised in that the control programme of the programmer (5) fundamentally corresponds to a triangular characteristic, in which the infusion rate increases from a base value to the highest value and then drops from this highest value with a predetermined functional dependence to the base value, particularly in a linear manner with a predetermined gradient or exponentially in the manner of an e-function.

7. Device as claimed in Claim 6, characterised in that the control programme of the programmer possesses a right-angled triangular characteristics (37, 38) in which case the area of a smallest elementary triangle whose short sides correspond to the smallest predeterminable infusion rate or time corresponds to one insulin unit, and the dose quantity of insulin units selected by the patient is specified by the programmer (5) as a multiple of the area of the elementary triangle, so that in the case of n-times the quantity of insulin units a triangle congruent with the elementary triangle is formed whose short sides are $\sqrt{n}$ longer in comparison to those of the elementary triangle (Fig. 3d).

8. Device as claimed in one of the preceding Claims, characterised in that the programmer (5) contains digital electronic stores.

9. Device as claimed in Claim 1, characterised in that the device housing (1, 22) of the control device (1) possesses setting up and display means (3, 4, 8, 23 to 26) for the input and display of the insulin units (IE) which are to be dispensed and of the absorbed carbohydrate content (BE).

10. Device as claimed in Claim 9, characterised in that the control device possesses an operating switch (28) which serves to change the output time of the insulin dispensation which is specified by means of the control programme of the programmer (5).

11. Device as claimed in Claim 1, characterised in that the control device (1) is accommodated in a device housing (1) which is separate from the micro-dose unit (II).

12. Device as claimed in Claim 11, characterised in that an electric line connection is provided for the transmission of the control signals between the control device (I) and the micro-dose unit (II).

13. Device as claimed in Claim 11, characterised in that the control signals are transmitted from the control device (I) to the micro-dose device (II) in wireless fashion by means of remote control (10, 12), preferably inductively.

**Revendications**

1. Dispositif pour l'injection pré-programmée d'insuline dans le corps humain ou animal, en diabétothérapie, constitué par une unité de microdosage (II) pour l'insuline et par un dispositif de commande (I) relié à celle-ci et comportant un générateur de programmes (5) pour l'unité de microdosage (II), caractérisé par le fait que le générateur de programmes est constitué par une mémoire à programmes associée au dispositif de commande (I), dans laquelle sont mémorisés simultanément plusieurs programmes de commande différents qui sont susceptibles d'être sélectionnés, à l'aide d'une unité d'entrée (2, 3), par le patient lui-même, grâce à l'introduction directe du nombre d'unités d'insuline (IE) qui correspond à des unités de pain (BE) absorbées pendant un repas, et qu'avec le processus de sélection au niveau de l'unité d'entrée (2, 3) est déclenché le déroulement, conforme au programme, de l'injection de l'insuline.

2. Dispositif suivant la revendication 1, caractérisé par le fait que sur le dispositif de commande (I), le patient peut régler, en plus de l'exécution (31 à 40), conformément au programme, d'une administration d'unités d'insuline (IE) choisies, un débit de base constant dans le temps (30) pour l'administration d'unités d'insuline (IE/h).

3. Dispositif suivant la revendication 1, caractérisé par le fait que simultanément lors du choix des unités d'insuline (IE) devant être administrées, se trouve appelé hors de la mémoire (5), qui constitue le générateur de programmes, un profil d'insuline fournie, qui est adapté exactement à la délivrance réelle d'insuline de la part d'un pancréas naturel.

4. Dispositif suivant la revendication 2 ou 3, caractérisé par le fait que le programme de commande du générateur de commande (5) présente la forme d'un rectangle (31 à 34), qu'un des côtés du rectangle peut être présélectionné par le patient en tant que débit d'injection ou durée d'injection et que l'autre côté du rectangle est déterminé, pour le maintien à une valeur constante du débit de dosage conformément au programme de commande prédéterminé, par le générateur de programmes (5) sous la forme d'une largeur variable ou d'une grandeur variable du rectangle (31 à 34) (figure 3b).

5. Dispositif suivant l'une des revendications 1 à 4, caractérisé par le fait que le programme de commande du générateur de programmes (5) possède la forme d'un rectangle, que les côtés latéraux d'un rectangle élémentaire, symbolisant une quantité délivrée d'insuline, correspondant au plus petit débit et à la plus petite durée d'injection pouvant être prédéterminés et que la quantité de dosage, choisie par le patient, en unités d'insuline, est déterminée par le générateur de programmes (5) sous la forme d'un multiple de la surface du rectangle élémentaire, de sorte qu'à une quantité n fois supérieure en unités d'insuline (IE) correspondent des débits et des durées respectifs plus importants d'injection (figure 3c).

6. Dispositif suivant l'une des revendications 1 à 5, caractérisé par le fait que le programme de commande du générateur de commandes (5) correspond sensiblement à la forme d'un triangle, que le débit d'injection augmente à partir d'une valeur de base jusqu'à la valeur maximale et ensuite retombe, depuis cette valeur, jusqu'à la valeur de base, avec une dépendance fonctionnelle prédéterminée, et notamment linéairement avec une pente prédéterminée ou bien exponentiellement à la manière d'une fonction e.

7. Dispositif suivant la revendication 6, caractérisé par le fait que le programme de commande du générateur de programmes possède la forme d'un triangle rectangle (37, 38), que la surface du plus petit triangle élémentaire, dont les côtés de l'angle droit correspondent au débit et à la durée d'injection les plus faibles pouvant être prédéterminés, correspond à une unité d'insuline et que la quantité de dosage, choisie par le patient, en unités d'insuline est déterminée par le générateur de programmes (5) sous la forme d'un multiple de la surface du triangle élémentaire, de sorte que, dans le cas d'une quantité n fois supérieure d'unités d'insuline, se trouve formé un triangle congruant avec le triangle élémentaire et dont les longueurs des côtés de l'angle droit sont supérieures du facteur $\sqrt{n}$ par rapport à ceux du triangle élémentaire (figure 3d).

8. Dispositif suivant l'une des revendications précédentes, caractérisé par le fait que le générateur de programmes (5) contient une mémoire électronique numérique.

9. Dispositif suivant la revendication 1, caractérisé par le fait que le boîtier (1, 22) de l'appareil du dispositif de commande (I) comporte des dispositifs de réglage et d'affichage (3, 4, 8, 23 à 26) pour l'introduction et l'affichage des unités d'insuline (IE) devant être administrées et des teneurs en hydrates de carbone (BE) absorbées.

10. Dispositif suivant la revendication 9, caractérisé par le fait que le dispositif de commande comporte un commutateur de manœuvre (28) permettant de modifier la durée, déterminée au moyen du programme de commande du générateur de programmes (5), de l'administration d'insuline.

11. Dispositif suivant la revendication 1, caractérisé par le fait que le dispositif de commande (I) est logé dans un boîtier (1) d'appareil séparé de l'unité de microdosage (II).

12. Dispositif suivant la revendication 11, caractérisé par le fait qu'il est prévu une liaison en forme de conducteur électrique pour la transmission des signaux de commande entre le dispositif de commande (I) et l'unité de microdosage (II).

13. Dispositif suivant la revendication 11, caractérisé par le fait que les signaux de commande sont transmis par le dispositif de commande (I) sans fil, au moyen d'une télécommande (10, 12), de préférence de façon inductive, au dispositif de microdosage (II).

8

FIG 1

Programm-geber I — 2, 3, 1, Anzeige 4, 8, 7, 5, Codierer 6, Sender 9, 10, Spule

Empfänger 11, 12, 13, Motor-verstärker 15, Decodierer 14, Batterie 20, Ventil 21, Vorrats-behälter 17, 18, 19, 16 Pumpenmotor II

FIG 2

28, 22, 24, 23, 26, 25, 27

0 002 776

FIG 3